## Europäisches Patentamt

## European Patent Office

## Office européen des brevets

(19)

(11) Publication number: **0 145 468**
**A2**

# EUROPEAN PATENT APPLICATION

(12)

(21) Application number: 84308537.4

(22) Date of filing: 07.12.84

(51) Int. Cl.⁴: **C 07 C 51/25,** C 07 C 57/04, C 07 C 57/08 // B01J23/44

(30) Priority: 07.12.83 US 559055
29.10.84 US 664562

(43) Date of publication of application: 19.06.85
**Bulletin 85/25**

(84) Designated Contracting States: **BE DE FR GB IT NL**

(71) Applicant: **Sun Tech, Inc., 1801 Market Street, Philadelphia, PA 19103 (US)**

(72) Inventor: **Suid, George, 201 W. Springfield Road, Springfield, PA 19064 (US)**
Inventor: **Lyons, James E., 211 Cooper Drive, Wallingford, PA 19086 (US)**

(74) Representative: **Laredo, Jack Joseph, Elkington and Fife High Holborn House 52/54 High Holborn, London, WC1V 6SH (GB)**

(54) Increased selectivities in the oxidation of olefins to alpha, beta-unsaturated carboxylic acids.

(57) Olefins such as propylene are oxidized directly to $\alpha,\beta$-unsaturated carboxylic acids such as acrylic acid with air or oxygen in an aqueous medium, employing an activated palladium-on-carbon catalyst, in the presence of certain surfactants such as sodium dodecyl sulfonate in combination with a co-surfactant such as n- or t-butyl alcohol. The catalyst pretreatment consists of reducing it with propylene at temperatures of at least about 60°C for at least about 10 minutes in the substantial absence of oxygen.
In a like manner isobutylene may be oxidized to methacrylic acid, while butene-1 may be oxidized to crotonic acid.

EP 0 145 468 A2

- 1 -

INCREASED SELECTIVITIES IN THE OXIDATION OF OLEFINS TO $\alpha$,$\beta$-UNSATURATED CARBOXYLIC ACIDS

## BACKGROUND OF THE INVENTION

### FIELD OF THE INVENTION

This invention relates to a process for the oxidation of olefins to form $\alpha$,$\beta$-unsaturated carboxylic acids. More particularly, this invention relates to an improved method for the oxidation of propylene to acrylic acid in the presence of a novel olefin-activated palladium

catalyst and a surfactant system comprising a combination of a surfactant and a co-surfactant.

In a like manner, isobutylene and butene-1 may be oxidized respectively to methacrylic acid and crotonic acid.

## DESCRIPTION OF THE PRIOR ART

The oxidation of propylene to acrylic acid in one step employing a palladium metal catalyst supported on carbon black is described in U.S. Patent 3,624,147. However, this process is characterized by yields of 60% or less, based on the amount of propylene converted, operating temperatures generally in excess of 90°C, and elevated pressures. Moreover, substantial amounts of $CO_2$ are reported as undesired by-products, as well as low reaction rates.

A similar process is reported in J. Catal., 173 (1972) by Sieyama et al., in which palladium black and palladium-activated charcoal were employed for converting propylene to acrylic acid. However, only a stoichiometric, non-catalytic conversion, based on the palladium metal, is taught, thus providing an even less effective method than in the above U.S. patent.

In addition, several patents describe conventional methods for the preparation of supported palladium metal catalysts by the reduction of, for example, palladium salts using such reducing agents as hydrogen, lower alcohols, hydrazine, or various olefins. See, for example, U.S.

0145468

Patents 3,275,680 to Holzrichter, or 4,435,598 to Hinnenkamp which teach reducing palladium salts with hydrogen or hydrazine. U.S. Patent 4,016,200 to Onoda likewise teaches that a palladium compound can be reduced to palladium metal, using formalin, hydrazine, hydrogen, methanol or olefins such as ethylene, propylene, or butene as reducing agents. Similarly, U.S. Patent 3,970,713 to Scharfe teaches the reduction of palladium and other metal salts to a metal catalyst, again using hydrogen, alcohols, olefins, etc. as reducing agents. However, none of these references teaches the preparation of a highly activated species of palladium metal which has been activated with an olefin under unique time and temperature conditions, or that such a catalyst is surprisingly effectively in a process for the oxidation of olefins to $\alpha,\beta$-unsaturated acids under more moderate operating conditions than have heretofore been possible. Finally, F. R. Hartley, "The Chemistry of Platinum and Palladium", Wiley and Sons, pp. 386-390, and 412-417 (1973) disclose the formation of a complex of ethylene with a palladium chloride to provide a palladium$^{+2}$ metal catalyst. However, as will be described below, the use of ethylene, or chlorides, and the formation of palladium$^{+2}$ metal catalysts have been found to deactivate the catalyst of this invention for purposes of forming the desired products claimed herein.

In U.S. application (Docket No. 83-046 CIP-1), referred to above, which is incorporated herein by reference in its entirety, and filed simultaneously herewith, there is described an improved method for the oxidation of propylene directly to acrylic acid with air or oxygen in an aqueous medium under moderate reaction conditions employing a supported palladium catalyst which has been pretreated by activating it with a

$C_3$-$C_6$ olefin, preferably propylene, at temperatures of at least about 60°C for at least about 10 minutes. The oxidation reaction is then continued in the presence of the pretreated catalyst at temperatures of at least about 25°C, whereby acrylic acid is recovered at high rates and selectivities as contrasted with the above prior art.

It is thus an object of the present invention to provide a further improvement of the afore-described olefin oxidation process, using certain surfactant systems, as set forth hereinbelow.

It is a further object of this invention to oxidize isobutylene to methacrylic acid and butene-1 of crotonic acid in the same manner.

## SUMMARY OF THE INVENTION

In accordance with the present invention, it has now been found that the selectivities of olefins such as propylene to form $\alpha$, $\beta$-unsaturated carboxylic acids such as acrylic acid may be further enhanced over those obtained by the use of an olefin-activated palladium catalyst supported on carbon or alumina, as described in copending application (Docket No. 83-046 CIP-1) and below, when the oxidation is carried out in the presence of a surfactant system comprising a surfactant and co-surfactant. By carrying out the aforesaid oxidation in the presence of these surfactants, it has been discovered that selectivties to acrylic acid are obtained in the range of from about 88 to 94%, i.e., an enhancement of about 5-10% over the use of the activated catalyst alone.

This same catalyst system, in combination with these surfactants, is likewise effective in oxidizing isobutylene to methacrylic acid, and butene-1 to crotonic acid.

Thus, olefins having from three to about six carbon atoms may be employed in this invention.

## DESCRIPTION OF THE PREFERRED EMBODIMENTS

As described above, the present invention is directed to a further improvement in a novel process for the oxidation of certain olefins, principally propylene, to their corresponding acids in the presence of a pretreated supported palladium catalyst, said improvement comprising carrying out the oxidation with air or oxygen in an aqueous medium in the presence of a combined surfactant/co-surfactant system such as sodium dodecyl sulfate in combination with n- or t-butyl alcohol.

The general method of oxidizing propylene to acrylic acid is adequately described in the prior art and need not be described herein in detail. Suffice it to say that utilizing the catalyst prepared by the novel methods discussed in detail below, in combination with applicants' claimed surfactant system, the oxidation reaction of propylene to acrylic acid can then be uniquely carried out at temperatures in the range of from about 25 to 125°C, and at pressures of 1 to 100 atm. Preferably, temperatures of from 25 to 85°C, and pressures of from 1 to 10 atm may be employed, as contrasted with the much more rigorous conditions employed in U.S. 3,624,147. As a consequence of this combined catalyst and surfactant system, selectivities, and thus yields, are significantly increased, over use of the catalyst alone, as shown in the examples below.

In one preferred embodiment of this process, in order to increase the reaction rate and at the same time reduce the reactor volume, it has been found to be advantageous that the reaction be carried out in a trickle bed reactor in which the liquid reaction medium is allowed to pass downward over a fixed catalyst bed and the acrylic acid product recovered at the bottom. Alternatively, the oxidation reaction can be carried out using an ebulating bed of catalyst while circulating gases and solvent.

The starting material from which the catalyst of this invention is prepared may be any finely divided palladium in the metallic state, on a support such as carbon or, less preferred, alumina, as for example a commercially available 5%, 10%, or 20% palladium on carbon available from standard catalyst manufacturers such as Engelhard Industries or Johnson Mathey, Inc. By the terms "palladium metal catalyst" or "palladium in the metallic state" is meant those palladium catalysts which have been prepared from their salts by known reduction means either commercially or as shown, for example, by Scharfe et al, U.S. Patent 3,970,713, or Holzrichter et al, U.S. Patent 3,275,680, but which have subsequently been exposed to the atmosphere in normal process procedures. While applicants do not wish to be bound by any particular theories, it is believed that in the normal course of handling and using the reduced catalysts of the prior art subsequent to reduction of the palladium, a certain proportion of the palladium surface species, by virtue of exposure to the atmosphere, becomes oxidized. It is this air-exposed palladium catalyst which is now being employed as the starting material in the preparation of applicants' novel olefin-activated catalyst. (By "surface species", as recognized by those skilled in the catalyst art, is meant any species of palladium found at the surface of the catalyst per se.)

Again, while applicants do not wish to be bound by any particular theory, it is believed that when this partly oxidized palladium surface, as described above, is contacted with propylene in accordance with applicants' invention, it is first converted to highly active palladium metal sites having zero valence, and it is with these sites that the propylene then forms the novel surface-active species which is the activated catalyst of this invention.

As evidence that the commercially-reduced palladium, for example, has been reoxidized under normal handling and exposure to air, it has been found that in the course of preparing the novel activated catalyst of this invention, starting, e.g., with a commercially reduced palladium metal catalyst, under oxygen-free conditions as described below, two parts propylene employed in activating the catalyst result in the formation of one part acetone and one part active catalyst species.

In preparing the activated oxidation catalyst used in this invention by treating a carbon-or alumina-supported palladium metal catalyst as defined above with propylene or like olefins, it is essential that this

-8-

0145468

activation treatment be carried out at temperatures of at least about 60°C, up to 150°C, preferably about 65 to 95°C, for a period of at least about 10 minutes to about 120 minutes, preferably at least about 30 to 60 minutes, under oxygen-free conditions as described below. This is generally carried out at pressures of at least about 1 atmosphere, up to about 100 atmospheres of propylene, although about 2-20 atmospheres is preferred. When these catalysts are thus activated, palladium-on-carbon, for example, which was otherwise far less reactive at temperatures below about 60°C for purposes of oxidizing propylene is now surprisingly active at temperatures of about 25°C or above. Moreover, as aforestated, the selectivities to acrylic acid are significantly improved by this treatment which is further enhanced by the use of applicants' defined surfactant system. Thus, by the term "activated palladium metal catalyst" is meant, for purposes of this invention, a catalyst prepared in accordance with the above method, and which can oxidize propylene to acrylic acid more rapidly and at lower temperatures than known supported palladium catalysts.

During the preparation of the catalyst, as stated above, it is necessary for purposes of deriving maximum activity from the catalyst that the activation be carried out in the substantial absence of oxygen, and preferably under essentially oxygen-free conditions. While the presence of small amounts of oxygen, to an extent which can be readily determined by those skilled in the art, can still result in a catalyst which performs under somewhat more mild conditions than the commercial catalysts described above, the full benefits of the present invention are derived from activating the catalyst under conditions which are as

0145468

oxygen-free as can be obtained, at least within the standards of commercial feasibility.

These oxygen-free conditions can be achieved by known means, for example by using deaerated water or solvent, and pure olefin gas, during the activation of the catalyst. Deaeration can be readily achieved by placing the liquid under vacuum until it boils, or by bubbling the desired olefin through the liquid for a period of time until no more oxygen is displaced. The pure olefin can be obtained commercially in various grades such as chemical purity grade, research purity grade, or polymer grade, the latter two being preferred because of their higher purity of over about 99.7%. (The latter two are available, for example, from Matheson, Division of Searle Medical Products, and Sun Co., respectively.)

Once applicants' catalyst is formed, it is preferable that at least a slight excess of olefin be present at all times to prevent any deactivation, and that desirably during the oxidation step, oxygen in the reactor be maintained in no greater than the stoichiometric amounts needed for the oxidation of the olefin to acrylic acid. It will also be understood that in preparing the catalyst of this invention, the presence of those metals or metal salts which might poison or alter the catalyst should be avoided, for example iron, manganese, copper and rhodium salts; chlorides, benzoquinone, the oxidized form of heteropoly acids, as well as any other agents which would oxidize palladium to palladium$^{+2}$. Other such deleterious materials can be routinely determined by those skilled in the art. For example, in addition, it has been found that such

0145468

materials as amines, hydrazine, and ethylene should be avoided as deleterious when preparing and using the catalyst of this invention. Moreover, it has been found that attempts to use hydrogen to prepare this catalyst may result in explosions when the catalyst is then exposed to $O_2$-propylene mixtures, and should also be avoided.

While the catalyst of the invention may be prepared separately and maintained in an active state if kept in an oxygen-free atmosphere, more conveniently the preparation is carried out in the same reactor used for the propylene oxidation. This may conveniently be achieved, for example by adding a commercially available finely divided palladium on activated carbon to an aqueous medium in a sealed reactor, flushing the system with propylene gas, and then heating the mixture under propylene pressure until the desired temperature for preparation of the catalyst is reached, at which time the mixture is stirred for at least 30 minutes at that temperature, again, in the absence of oxygen, and desirably in the presence of a slight excess of olefin.

After the preparation of the catalyst, the propylene may be replaced by a mixture of propylene and oxygen, desirably with oxygen being present in approximately stoichiometric amounts to avoid deactivation of the catalyst, the surfactant system added to the liquid medium, if not already present, and the oxidation reaction carried out at pressures of from about 1 to 10 atmospheres. The pressure may be maintained by the further addition of the gas mixture from time to time until the desired propylene conversion is achieved. Air may be used in place of oxygen, in which case the amount of propylene must be adjusted proportionately.

0145468

While the activating agent for the catalyst is preferably propylene, if desired there may instead be employed other light olefins having an allylic hydrogen and containing from 3-6 carbon atoms, preferably those corresponding to the olefins to be oxidized. Most preferred, in addition to propylene, are butene-1, butene-2 or isobutylene.

The olefin-activated catalyst maintains its activity over long periods of time as long as at least small amounts of an acceptable olefin are present. Thus, it has been found beneficial to run the reaction by constantly sparging the propylene/oxygen or air reaction mixture through the aqueous solution. In this way, the propylene is kept in excess and the catalyst remains highly active, thereby maintaining high selectivities and other advantages noted above.

When carrying out the oxidation in a batch-wise manner the ratio of catalyst to reaction medium is desirably in the range of about 0.05-5.0 gram atoms of palladium per liter of reactant, and preferably about 0.1-1.0 gram atoms. In a continuous process utilizing, e.g., a fixed bed reactor, the reaction can be conducted effectively by varying the volume of reactants and contact time with the catalyst in a generally known manner to achieve the high yields and selectivities disclosed herein.

In general, any of a series of known sulfonate surfactants which increase propylene, isobutylene, or butene-1 solubility, or allow phase transfer catalysis or both, may be employed in this process. Good selectivities for acrylic acid, together with reduced carbon dioxide formation make this particular system a superior process for the oxidation of propylene to acrylic acid under mild conditions. When the surfactants are used in accordance with this improved process, selectivities of 88-94% are unexpectedly obtained, whereas in the absence of surfactants under the same conditions selectivities are typically 80-85%. The surfactant system may be added to the liquid medium at any point prior to oxidation, i.e. either before or after activation.

Although the preferred surfactant system is a combination of sodium dodecyl sulfate and a fatty alcohol co-surfactant such as n- and/or t-butyl alcohol, other surfactants such as the sodium salt of a $C_{14}$ - $C_{17}$ alkyl sulfonate of secondary alcohols (e.g. "HOSTAPUR" SAS-30N, SAS-60, or SAS-93 available from American Hoechst) may be employed instead, together with a suitable fatty alcohol. Other alcohol co-surfactants which may also be employed include isopropyl alcohol, n-propyl alcohol and sec.-butyl alcohol.

From the table below it can be seen that there is an optimum amount of surfactant above which reduced selectivities are observed. Thus, for example, when 2% sodium dodecylsulfonate and 2% alcohol are used, selectivities fall to about 70%. Therefore, the preferred amounts of surfactant and co-surfactant, based on the weight of aqueous medium, is in the range of from about 0.1 - 1.5% for each, and most preferably about 0.4 - 1.2% for each.

The following examples are by way of illustration of the invention.

EXAMPLES 1-10

In the following examples, 1-10, a number of reactions were run in accordance with the following general procedures:

One gram of 10% palladium metal on carbon (Engelhard Industries) was added to an 85 ml Fisher-Porter aerosol tube. Then 30 ml of deaerated distilled water was added and the Fisher-Porter tube was fitted to a pressure manifold. The surfactants, in the amount listed in the Table, were then added.

The mixture was flushed to 50 psi three times with pure propylene gas (research purity grade). It was then heated with stirring under 50 psi of this pure propylene until it reached the desired activation temperature where the mixture was stirred for at least about 10 minutes. The stirred mixture was then brought to the desired reaction temperature and the propylene was replaced with a gas mixture having the composition: 60% $O_2$/40% pure $C_3H_6$ to a total pressure of 100 psig. Reaction proceeded immediately in most cases and the pressure dropped. When the total pressure reached 80 psig the $O_2$/$C_3H_6$ gas mixture was admitted to bring the total pressure to 100 psig. This was repeated as often as necessary during the course of the run. After the determined reaction time the mixture was cooled, the gas captured and analyzed and the mixture filtered. The catalyst was washed with both organic and aqueous

0145468

solutions to remove small amounts of acrylic acid held on the surface. The filtrates were analyzed by standardized GC to determine the product composition.

The results, together with certain variations in the reaction conditions, are shown in Table I below. From these results it will be seen that the addition of surfactant systems enhance acrylic acid yields in oxidation over propylene-activated supported palladium catalysts.

## TABLE I
### GC Analysis of Liquid Phase[d]

| Example | Surfactant System[a] | Time (min) | Total[b] (PSIG) | Acrylic Acid (MMOLES) | Acrylic Acid % | Acetic Acid % | Acetone % | Others % | Amount $CO_2$ Milli-moles | % |
|---|---|---|---|---|---|---|---|---|---|---|
| 1 | None | 220 | 75 | 4.30 | 79.4 | 3.0 | 7.9 | 9.8 | 0.80 | 5.20 |
| 2 | 0.5% SDS 0.5% n-BA[c] | 194 | 61 | 5.34 | 87.9 | 3.8 | 4.5 | 4.0 | 0.71 | 3.57 |
| 3 | None | 390 | 115 | 7.74 | 84.9 | 7.8 | 4.3 | 3.1 | 1.16 | 6.80 |
| 4 | 0.5% SDS 0.5% n-BA | 436 | 106 | 8.00 | 88.8 | 5.5 | 2.4 | 3.3 | 1.44 | 8.04 |
| 5 | 0.5% SDS 0.5% t-BA | 436 | 79 | 5.63 | 88.0 | 5.9 | 2.7 | 3.3 | 1.11 | 5.84 |
| 6 | 0.5% SDS | 335 | 59 | 4.48 | 87.7 | 4.9 | 5.0 | 2.5 | 0.99 | 5.00 |
| 7 | 1% SDS 1% n-BA | 236 | 42 | 3.18 | 86.0 | 3.7 | 5.4 | 4.9 | 0.54 | 3.93 |
| 8 | 1% SDS 1% t-BA | 236 | 35 | 2.13 | 93.5 | tr | 4.9 | 1.6 | 0.34 | 2.13 |
| 9 | 2% SDS 2% t-BA | 287 | 62 | 3.99 | 72.0 | 2.1 | 5.8 | 20.1 | 0.85 | 4.74 |
| 10 | 2% SDS 2% n-BA | 287 | 56 | 4.26 | 68.5 | 1.9 | 2.8 | 26.8 | 0.71 | 3.58 |

a) SDS - sodium dodecyl sulfate
b) Total pressure drop over the total reaction time.
c) BA - butyl alcohol
d) Activation and reaction temperature = 65°C

## Example 11

When the reaction is run in accordance with the procedures of Example 8, except that the catalyst is activated for at least 30 minutes at 65°C prior to reaction, acrylic acid is obtained in greater than 94% yield by standardized glpc analysis.

## Example 12

When the reaction is carried out in accordance with the procedures of Example 8, except that isobutylene is substituted for propylene, methacrylic acid is formed in good yield, together with like amounts of methacrolein.

## Example 13

When the reaction is carried out in accordance with the procedures of Example 8, except that butene-1 is substituted for propylene, crotonic acid is formed in good yield.

CLAIMS

1.     A process for the preparation of $\alpha,\beta$ -unsaturated carboxylic acids which comprises first activating a supported palladium metal catalyst by contacting it with a $C_3-C_6$ olefin in a liquid medium at a temperature of at least about 60°C for at least about 10 minutes in the substantial absence of oxygen, and thereafter contacting said activated catalyst with said $C_3-C_6$ olefin admixed with air or oxygen in said liquid medium containing a surfactant system comprising a surfactant and co-surfactant in amounts of not more than 1.5% by volume of each, thereby oxidizing said olefin to the corresponding carboxylic acid.

2.     A process for the preparation of $\alpha,\beta$ -unsaturated carboxylic acids which comprises oxidizing a $C_3-C_6$ olefin admixed with air or oxygen in the presence of an activated palladium metal catalyst in a liquid medium containing a furfactant system comprising a surfactant and co-surfactant, in amounts of not more than about 1.5% by volume of each, wherein said catalyst is activated by contacting a supported palladium metal catalyst in said liquid medium with said $C_3-C_6$ olefin at temperatures of at least about 60°C for at least about 10 minutes in the substantial absence of oxygen.

3.     A process for the preparation of $\alpha,\beta$ -unsaturated carboxylic acids which comprises first activating a supported palladium metal catalyst by contacting it with a $C_3-C_6$ olefin in a liquid medium at a temperature of at least about 60°C for at least about 10 minutes in the substantial absence of oxygen, and thereafter contacting said activated catalyst with a different $C_3-C_6$ olefin admixed with air or oxygen in said liquid medium containing a surfactant system comprising a surfactant and co-surfactant in amounts of not more than about 1.5% by volume of each, thereby oxidizing said olefin to the corresponding carboxylic acid.

4.     A process for the preparation of $\alpha,\beta$-unsaturated carboxylic acids which comprises oxidizing a $C_3$-$C_6$ olefin admixed with air or oxygen in the presence of an activated palladium metal catalyst in a liquid medium containinga surfactant system comprising a surfactant and co-surfactant, in amounts of not more than about 1.5% by volume of each, wherein said catalyst is activated by contacting a supported palladium metal catalyst in said liquid medium with a different $C_3$-$C_6$ olefin at temperatures of at least about 60°C for at least about 10 minutes in the substantial absence of oxygen.

5.     The process of any of Claims 1 to 4, wherein the catalyst is activated in the essential absence of oxygen.

6.     The process of any of Claims 1 to 4, wherein the catalyst is maintained in its activated state by continuous contact with said olefin.

7.     The process of any of Claims 1 to 4, wherein the catalyst is maintained in its activated state by continuous contact with said olefin being oxidized.

8.     The process of any of Claims 1 to 7, wherein the catalyst is activated with said olefin under pressures of from about 1 to 100 atmospheres of said olefin.

9.     The process of any of Claims 1 to 8, wherein the catalyst is activated with said olefin at temperatures of from 60°C to 150°C. for at least about 10 to 120 minutes.

10.     The process of any of Claims 1 to 9, wherein the olefin is propylene and the carboxylic acid is acrylic acid.

11.     The process of any of Claims 1 to 9, wherein the olefin is isobutylene and the carboxylic acid is methacrylic acid.

12.     The process of any of Claims 1 to 9, wherein the olefin is butene-1 and the carboxylic acid is crotonic acid.

13.     The process of any of Claims 1 to 12, wherein the oxidation is carried out with stoichiometric amounts of olefin and oxygen needed to produce said $\alpha,\beta$ -unsaturated carboxylic acid.

14.     The process of any of Claims 1 to 13, wherein the support for the palladium metal is carbon or alumina.

15.     The process of any of Claims 1 to 14, wherein the oxidation is carried out at temperatures of at least about 25°C.

16.     A process according to any of Claims 1 to 15, wherein the surfactant system comprises a mixture of an alkali metal salt of $C_{14}$-$C_{17}$ alkyl sulfonate of secondary alcohols, and sec.-butyl alcohol.

17.     The process of any of Claims 1 to 16, wherein the surfactant system comprises sodium dodecyl sulfate and n- or t- butyl alcohol.

0145468

18.    The process of any of Claims 1 to 17, wherein
the amount of surfactant and co-surfactant in the liquid
medium is each in the range of from about 0.1 to 1.5%
by volume.